(19) Europäisches Patentamt
European Patent Office
Office européen des brevets

(11) Veröffentlichungsnummer: **0 091 045**
**B1**

(12) **EUROPÄISCHE PATENTSCHRIFT**

(45) Veröffentlichungstag der Patentschrift: **26.02.86**

(21) Anmeldenummer: **83103028.3**

(22) Anmeldetag: **26.03.83**

(51) Int. Cl.⁴: **C 07 D 207/38**, C 07 D 401/04, C 07 D 409/04, A 61 K 31/40 // C07D207/36, (C07D401/04, 213:57, 207:38), (C07D409/04, 333:24, 207:38)

(54) **2-Pyrrolin-3-carbonitril-Derivate, Verfahren zu deren Herstellung und diese enthaltende Arzneimittel.**

(30) Priorität: **03.04.82 DE 3212591**

(43) Veröffentlichungstag der Anmeldung: **12.10.83 Patentblatt 83/41**

(45) Bekanntmachung des Hinweises auf die Patenterteilung: **26.02.86 Patentblatt 86/9**

(84) Benannte Vertragsstaaten: **AT BE CH DE FR GB IT LI LU NL SE**

(56) Entgegenhaltungen:
**CH - A - 313 054**

**AUST. J. CHEM., Band 27, 1974, Seiten 2229-2241, J.W. DUCKER et al.: "The reactions of malononitrile with alpha-diketones and related studies"**
**SYNTHESIS, 1980, Seiten 900-901, Georg Thieme Verlag, Stuttgart, DE., S. AKABORI et al.: "one-step synthesis of 4-cyano-3,3-diaryl-5-methyl-2-oxo-2,3-dihydropyrroles through the benzilic acid rearrangement"**

(73) Patentinhaber: **GÖDECKE AKTIENGESELLSCHAFT, Salzufer 16, D-1000 Berlin 10 (DE)**

(72) Erfinder: **Barth, Hubert, Dr., Rosenweg 60, D-7830 Emmendingen (DE)**
Erfinder: **Fritschi, Edgar, Dr., Am Scheuerwald 2, D-7811 St. Peter (DE)**
Erfinder: **Ganser, Volker, Dr., Sulzburger Strasse 22, D-7800 Freiburg (DE)**
Erfinder: **Hartenstein, Johannes, Dr., Fohrenbühl 23, D-7801 Stegen-Wittental (DE)**
Erfinder: **Herrmann, Manfred, Dr., Wolfweg 25, D-7811 St. Peter (DE)**
Erfinder: **Satzinger, Gerhard, Dr., Im Mattenbühl 7, D-7809 Denzlingen (DE)**

**EP 0 091 045 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegegenstand der Erfindung sind substituierte 5-Oxo-2-pyrrolin-3-carbo-nitrile der allgemeinen Formel I

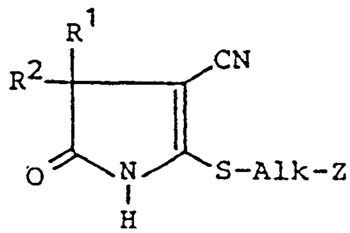

in welcher $R^1$ und $R^2$ gleich oder verschiden sind und einen unsubstituierten oder durch bis zu zwei Halogenatome-, $C_{1-4}$ Alkoxygruppen, $C_{1-4}$ Dialkylaminogruppen oder Trifluormethylgruppen substituierten Phenyl-, Pyridyl- oder Thienylring, Alk eine geradkettige oder verzweigte $C_{1-4}$ Alkylenkette und Z Wasserstoff darstellt, wobei für den Fall, daß Alk eine geradkettige Alkylenkette bedeutet, Z auch ein Aminogruppe der allgemeinen Formel II bedeuten kann.

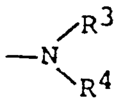

in welcher $R^3$ und $R^4$ gleich oder verschieden sind und ein Wasserstoffatom oder einen Methyl- Äthyl-, Propyl- oder Isopropylrest bedeuten oder zusammen mit dem Stickstoffatom an das sie gebunden sind, eine Pyrrolidino-, Piperidino-, Morpholino- oder Piperazinogruppe darstellen können,

sowie deren pharmakologisch verträgliche Salze.

Bevorzugt sind Verbindungen, in denen $R^1$ und $R^2$ gleich oder verschieden sind und einen Phenyl-, 4-Chlorphenyl-, 4-Dimethyl-aminophenyl-, 4-Fluorphenyl-, 4-Methyxypenyl-, 2-Pyridyl-, 3-Pyri-dyl-, 2-Thienyl-, und 3-Trifluormethylphenylrest bedeuten und Alk-Z eine Methyl-. Ethyl-, Isopropyl-, Aminoethyl-, Aminopropyl-. Diethylaminoethyl-. Äthylaminoethyl- oder Diethylaminopropylgruppe darstellt.

Die Verbindungen der Beispiele 4a: (2-(2-Diethylaminoethylthio)4-(4-dimethylaminophenyl)-5-oxo-4-(3-pyridyl)-2-pyrrolin-3-carbonitril). 4i: 4-(4-Dimethylaminophenyl)-2-methylthio-5-oxo-4-(3-pyridyl)-2-pyrrolin-3-carbonitril und 4k: 2-(3-Diethylaminopropylthio)-4-(4-dimethylaminophenyl)-5-oxo-4-(3-pyridyl)-2-pyrrolin-3-carbonitril werden besonders bevorzugt.

Ein weiterer Gegenstand der Erfindung ist ein chemisch eigenartiges Verfahren zur Herstellung von Verbindungen der allgemeinen Formel I, das dadurch gekennzeichnet ist, daß man eine Verbindung der allgemeinen Formel III

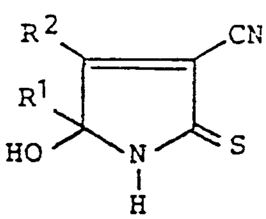

in welcher $R^1$ und $R^2$ die oben genannte Bedeutung haben, in einem Lösungsmittel mit einer Base zu einer Verbindung der allgemeinen Forme IV

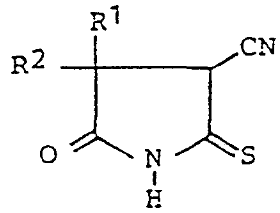

in welcher $R^1$ und $R^2$ die oben genannte Bedeutung haben. umlagert und diese anschließend mit einer Verbindung der allgemeinen Formel V

X-Alk-Z

in welcher Z und Alk die obengenannte Bedeutung haben und X eine reaktive Ester oder Ethergruppe darstellt, umsetzt und die erhaltene Verbindung der allgemeinen Formel I anschließend gegebenenfalls in deren pharmakologisch verträglichen Salze überführt.

Bei der Umsetzung von Verbindungen der allgemeinen Formel III mit Basen, die bevorzugt in einem polaren Lösungsmittel wie z.B. einem niedrig siedenden Alkohol bei Rückflußtemperatur durchgeführt wird, findet eine Umlagerung unter Bildung von Verbindungen der allgemeinen Formel IV statt.

Als Lösungsmittel kommen insbesondere polare Lösungsmittel infrage. Bevorzugt werden niedere Alkohole wie z.B. Methanol, Ethanol oder n-Butanol verwendet. Die Umlagerungsreaktion erfordert die Anwesenheit starker Basen. Bevorzugt werden als Basen Kaliumhydroxid, Kaliumcarbonat. Natriummethanolat, Natriumethanolat oder Kalium-tert.-butylat eingesetzt.

Die Reaktionszeiten betragen in den meisten Fällen 5 bis 30 Minuten. Der Reaktionsverlauf kann ander deutlich sichtbaren Aufhellung der anfangs meist tief orange bis gelb gefärbten Reaktionsmischung gut verfolgt werden. Die Umlagerungsprodukte können nach vorsichtigem Ansäuern der Reaktionsmischung isoliert und als solche zur Alkylierung eingesetzt werden.

Vorzugsweise werden die Umlagerungsprodukte jedoch nicht isoliert, sondern in einer Art Eintopfreaktion direkt mit einem geeigneten reaktiven Ester oder Ether der Formel V umgesetzt. Als reaktive Estergruppen werden erfindungsgemäß Halogenatome, Alkylsulfat-, Alkyloder Arylsulfonatgruppen verstanden. Hierfür eignen sich insbesondere die ent sprechenden Halogenide. Sulfate, p-Toluolsulfonate oder p-Bromtoluolsulfonate. Dialkylaminoalkylhalogenide werden bevorzugt in Form ihrer hydrocloride oder -bromide eingeset zt. In diesem Fall verwendet für die Alkylierungsreaktion 2 Mol äquivalente Base. Bei Verwendung eines Trialkyloxoniumsalzes bedeutet X eine Dialkylethergruppe.

Die Reaktionsprodukte der allgemeinen Formel I kristallisieren nach Filtration vom ausgefallenen Alkalihalogenid direkt aus dem Filtrat aus oder werden nach Einengen der Reaktionsmischung und Verteilen des Rückstands zwischen einem mit Wasser nicht michbaren organischen Lösungsmittel, wie z.B. Methylenchlorid, und Wasser nach Einengen der organischen Phase und Kristallisieren aus einem geeigneten Lösungsmittel erhalten.

Schwer kristallisierende Basen werden in geeignete Säureadditionssalze überführt. Als Säureadditionssalze kommen die Salze organischer und anorganischer Säuren, wie z.B. Salzsäure, Schwefelsäure, Phosphorsäure, Bromwasserstoffsäure, Essigsäure. Trifluoressigsäure. Weinsäure, Michsäure, Zitronensäure. Äpfelsäure, Salicylsäure. Ascorbinsäure, Malonsäure, Maleinsäure oder Bernsteinsäure infrage. Die Salze werden in an sich bekannter Weise durch Umsetzung der Basen mit entsprechenden organischen oder anorganischen Säuren hergestellt.

Die als Ausgangsmaterial dienenden Verbindungen der allgemeinen Formel III erhält man durch Reaktion entsprechend substituierter und bekannter Benzile mit 2-Cyanthioacetamid in Gegenwart katalytischer Mengen einer Base wie z.B. Triethylamin oder Piperidin bei Raum- oder Rückflußtemperatur. Als Lösungsmittel eignen sich polare Lösungsmittel, bevorzugt Chloroform, Dichlormethan, Methanol oder Ethanol. Die Reaktionszeiten betragen 2 bis 20 Stunden.

Die meist gelb bis orange gefärbten Verbindungen der allgemeinen Formel III fallen im Verlauf der Reaktion in der Regel als schwerlösliche Niederschläge aus und können in an sich bekannter Weise durch Absaugen, Auswaschen und Trocknen isoliert werden oder sie werden nach Entfernen des Lösungsmittels aus dem Rückstand erhalten. Bei Verwendung unsymmetrischer Benzile können isomere Produkte der allgemeinen Formel III entstehen, bei denen $R^1$ und $R^2$ vertauscht sind. Die nachfolgende Umlagerung und Alkylierung führt jedoch zu demselben Endprodukt der allgemeinen Formel I. Die verwendeten Benzile sind bekannt oder können

nach bekannten Vorschriften und Methoden. z.B. durch Oxidation geeigneter Benzoine mit Kupfer(II)salzen hergestellt werden (vgl. hierzu Organic Reactions, Band IV, Kapitel 5, S. 269. 1948. HOUBEN WEYL, Band 7/2a. S. 653 und S. 751).

Die Verbindungen der allgemeinen Formel I zeigen Aktivitäten, welche ein neues Prinzip in der Therapie der Entzündung bzw. der Ulkus-Krankheit darstellen.Bisher zeigten entzündungshemmende Substanzen vom Typ der nicht-steroidalen Antiphlogistika häufig ulzerogene Nebenwirkungen. Im Gegensatz dazu sind die erfindungsgemäßen Substanzen-bei ausgezeichneter Verträglichkeit und sehr guter Entzündungshemmung überraschend ulkusprotektiv.

Somit sind die Verbindungen der allgemeinen Formel I zur Therapie von Entzündungen geeignet, ohne die bei allen bisher bekannten Verbindungen auftretenden gastrointestinalen Nebenwirkungen zu zeigen. Sie sind aber auch zur Therapie der Ulkus-Krankheit geeignet, da gerade die ulkusprotektive Wirkung günstig durch die entzündungshemmende Wirkung ergänzt wird.

Die Dosierung beim Menschen dürfte bedingt durch die gute Verträg lichkeit der Verbindungen und abhängig vom Schweregrad der Erkrankung bei etwa 50 - 100 mg bei oraler bzw. parenteraler einmaliger Verabreichung liegen. Die Tagesdosis beträgt entsprechend etwa 100 - 2000 mg.

Die erfindungsgemäßen verbindungen der allgemeinen Formel i können in flüssiger oder fester Form oral oder parenteral appliziert werden. Als Injektionslösung kommt vor allem Wasser zur Anwendung, welches die bei Injektionslösungen üblicen Zusätze wie Stabilisierungsmittel, Lösungsvermittler oder Puffer enthält.

Derartige Zusätze sind z.B. Tartrat- und Citrat-Puffer, Äthanol, Komplexbildner (wie Athylendiamin tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat,tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Ein weiterer Gegenstand der Erfindung sind demgemäß Arzneimittel, gekennenzeichnet durch einen Gehalt an Verbindungen gemäß Anspruch 1 nebst üblichen Zusatz- und/oder Trägerstoffen.

Der folgende Versuchsbericht erläutert die Wirkung der Verbindungen gemäß der allgemeinen Formel I:

**Untersuchung der akuten Toxizität**

Methode:

Die Bestimmung der akuten Toxizität wurde an männlichen Mäusen (NMRI) mit einem Körpergewicht von 18 bis 23 g durchgeführt. Alle Versuchstiere wurden 20 Stunden vor Versuchsbeginn nüchtern gesetzt. Wasser stand ad libitum zur Verfügung. Zu jeder Dosiennngsgruppe gehörten 4 Tiere. Die Dosenfolge war logarithmisch. Die Prüfsubstanzen wurden als Suspension in 1 %igem Tragantschleim intragastral verabreicht. Das Applicationsvolumen betrug 20 ml/kg Körpergewicht. Die Tiere wurden insgesamt 7 Tage beobachtet.

Ergebnisse:

In der folgenden Tabelle sind die $LD_{50}$-Werte nach 7-tägiger Beobachtung der Tiere wiedergegeben.

Akute Toxizität an Mäusen

| Beispiel Nr. | Applikations-art | $LD_{50}$ mg/kg |
|---|---|---|
| 4 a | intragastral | 800 |
| 4 b | intragastral | 400 |
| 4 d | intragastral | 1600 |
| 4 e | intragastral | > 1600 |
| 4 g | intragastral | 1600 |
| 4 i | intragastral | > 1600 |
| 4 k | intragastral | 300 |
| 6 c | intragastral | > 1600 |
| 6 f | intragastral | > 1600 |

**Untersuchung der antiphlogistischen Wirkung am Carrageeninödem der Ratte**

Methode:

Versuchstiere waren männliche, 20 Stunden nühchtern gesetzte Ratten (SIV 50) mit einem Körpergewicht von 110 bis 160 g. Wasser stand ad libitum zur Verfügung. Nach Ermittlung des Ausgangswertes des Pfotenvolumens der rechten Hinterextremität wurden die Prüfsubstanzen intragastral als Suspension in 1 %igem Tragantschleim verabreicht. Pro Dosis wurden 10 Tiere verwedet. Das Applikationsvolumen betrug 20 ml/kg Körpergewicht. Das pfotenvolumen wurde mittels Quecksilberverdrängung gemessen. 60 Minuten nach intragastraler Verabreichung der Prüfsubstanzen wurde 0,1 ml einer 1 %igen Carrageeninlösung subplantar in die rechte Hinterpfote injiziert.

1 und 3 Stunden nach Injektion des Carrageenis wurde das Pfotenvolumen der ödematösen pfote gemessen. Die Zunahme des Pfotenvolumens der substanzbehandelten Tiere nach 3 Stunden wurde mit derjenigen bei den Kontrolltieren verglichen und die Hemmung des Pfotenödems durch die Prüfsubstanz in Prozent davon berechnet.

Ergebnisse:

Antiphlogistische Wirkung am Carrageeninödem der Ratte

| Beispiel Nr. | Dosis mg/kg intragastral | Hemmung des Pfotenvolumens in % |
|---|---|---|
| 4 a | 50<br>100 | 59<br>77 |
| 4 d | 50<br>125 | 26<br>83 |
| 4 e | 25<br>50 | 37<br>70 |
| 4 g | 50<br>100 | 25<br>61 |
| 6 c | 125<br>250 | 6<br>45 |
| 6 f | 125<br>250 | 24<br>45 |

Die in der Tabelle aufgefürten Verbindungen zeigen bei gleichzeitig guter oraler Verträglichkeit bemerkenswerte antiphlogistische Eigenschaften, wobei die beiden wirksamsten Vertreter Beispiel 4 a und Beispiel 4 e sind.

**Untersuchung des Einflusses auf die Magensaftsekretion bei Ratten**

Methode:

Nach der von H. Shay et al. (Gastroenterology 5, 43-61, 1945) beschriebenen Methode wurden männliche Ratten (SIV 50) mit einem Körpergewicht von 160 bis 200 g 48 Stunden vor Versuchsbeginn nüchtern gesetz. Wasser stand den Tieren ad libitium zur Verfügung. Die Prüfsubstanzen wurden als Suspension in 1 %igem Tragantschleim intragastral verarbrecht. Das Applikationsvolumen betrug 10 ml/kg Körpergewicht. Pro Versuchsgruppe wurden 8 Tiere verwendet. Eine Stunde nach Substanzapplikation wurde in leichter Äthernarkose eine Pylorus-Lignatur gelegt. Vier Stunden nach diesem Eingriff wurden die Tiere getötet. Der exzstirpierte Magen wurde entlang der Curvatura major eröffnet, das Volumen des Sekretes gemessen und in Relation zu den Daten der Kontrollgruppe gesetzt. Ergebnisse: Einfluß auf die Magensaftsekretion bei Ratten

| Beispiel Nr. | Dosis mg/kg intragastral | Hemmung der Magensaftsekretion in % |
|---|---|---|
| 4 a | 62,5<br>125<br>250 | 45<br>64<br>73 |
| 4 b | 50<br>100<br>200 | 24<br>63<br>76 |
| 4 d | 125<br>250 | 48<br>76 |

Wie die Tabelle zeigt, ergibt sich für die dort aufgeführten verb dungen im Versuch nach Shay bei Ratten eine ausgeprägte, dosisabhängige Hemmung der Magensaftsekretion.

**Untersuchung der ulcusprotektiven Wirkung am durch Indometazin induzierten Magenulcus der Ratte.**

Methode:

Versuchstiere waren männliche, 20 Stunden nüchtern gesetzte Ratten (SIV 50) mit einem Körpergewicht von 170 bis 220 g. Wasser stand ad libitum zur Verfügung. Den Tieren wurde simultan 40 mg/kg Indometazin und die zu prüfende Substanz als Suspension in 0,8 %igem Methocel intragastral verabreicht. Die Kontrolltiere erhielten nur die entsprechende Dosis Indometazin. Pro Versuchsgruppe wurden 10 Tiere verwendet. Das Applikationsvolumen betrug 10 ml/kg Körpergewicht. 5 Stunden nach Application wurden die Tiere getötet. Nach Entnahme der Mägen und Eröffnung an der Curvatura major wurden diese mittels einer Stereolupe auf das Vorhandensein von Ulcera untersucht. Die Beurteilung der Ulcera erfolgte nach einem von M. Chaumontet et al. (Arzneim.-Forsch. 28, 2119 1978) vorgeschlagenen Punkteschema. Hieraus wurde der Ulcusindex berechnet. Der Ulcusindex der mit Indometazin und Prüfsubstanz behandelten Tiere wurde mit demjenigen der nur mit Infometazin behandelten Kontrolltiere verglichen und daraus die Ulcusshemmung in Prozent berechnet.

Ergebnisse: Ulcusprotektive Wirkung am durch Indometazin induzierten Magenulcus der Ratte.

| Beispiel Nr. | Dosis mg/kg intragastral | Ulcushemmung in % |
|---|---|---|
| 4 a | 125 | 84 |
| 4 i | 50 | 88 |
| 4 k | 50 | 33 |
| | 100 | 94 |

Die in der Tabelle aufgeführten Verbindungen besitzen bemerkenswerte ulcusprotektive Eigenschaften, wobei sich für Beispel 4 i aufgrund der guten, Verträglichkeit ein besonders günstiger therapeutisher Quotient ergibt.

Die folgenden Beispiele dienen zur Erläuterung des Herstellungsverfahrens für die erfindungsgemäßen Substanzen.

**Beispiel 1**

**2-(2-Diethylaminoethylthio)-4,4-diphenyl-5-oxo-2-pyrrolin-3-carbonitril**

10,2 g 5-Hydroxy-4,5-diphenyl-2-thioxo-3-pyrrolin-3-carbonitril und 9,7 g wasserfreies Kaliumcarbonat werden 20 Minuten in 250 ml Ethanol unter Rückfluß erhitzt. Dann tropft man eine Lösung von 6,0 g 2-Diethylaminoethylchlorid-Hydrochlorid in 50 ml Ethanol innerhalb von 10 Minuten hinzu und kocht 1 Stunde weiter. Man gibt die Reaktionsmischung auf Eis. filtriert den erhaltenen Niederschlag ab und kristallisiert aus Essigester um. Man erhält 11.9 g (87 %) 2-(2-Di-ethylaminoethylthio)-4.4-diphenyl-5-oxo-2-pyrrolin-3-carbonitril in Form farbloser Kristalle: Fp. 156-157° C.

Das als Ausgangsprodukt verwendete 5-Hydroxy-4,5-diphenyl-2-thioxo-3-pyrrolin-3-carbonitril wird wie folgt hergestellt:

Eine Mischung von 60.0 g Benzil, 28,6 g Cyanthioacetamid, 500 ml Methanol und 20 Tropfen Piperidin wird 5 Stunden bei Raumtemperatur gerührt. Danach zieht man das Lösungsmittel am Rotationsverdampfer ab und kristallisiert den Rückstand aus Toluol. Nach Trocknen im Vakuum bei 0,1 Torr und 80° C erhält man 80.1 g gelbe Kristalle Fp. 195° C (Zers.).

In analoger Weise erhält man folgende Verbindungen:

4,4-Diphenyl-5-oxo-2- (2-piperidinoethylthio -2-pyrrolin-3-carboni-Fp. 177 - 178° C.

2-(2-Dimethylaminopropylthio)-4,4-diphenyl-5-oxo-2-pyrrolin-3-carboni-tril: Fp. 250° C (Zers.)

4,4-Diphenyl-2-ethylthio-5-oxo-2-pyrrolin-3-carbonitril: Fp. 180 C.

4, 4-Diphenyl-2- ( 2-morpholinoethylthio-5-oxo-2-pyrrolin-3-carbonitril Fp 170° C.

**Beispiel 2**

**4-(4-Chlorphenyl)-4-(4-dimethylaminophenyl)-2-ethylthio-5-oxo-2pyrrolin-3-carbonitril**

25,9 g 4-(4-Chlorphenyl)-5-(4-dimethylaminophenyl)-5-hydroxy-2-thioxo-3-pyrrolin-3-carbonitril und 19.3 g wasserfreies Kaliumcarbonat werden 40 Minuten in 400 ml Ethanol erhitzt. Dann tropft man eine Lösung von 7,6 g Ethylbromid in 40 ml Ethanol innerhalb von 15 Minuten hinzu und kocht 1 Stunde weiter. Nach dem Einengen der Reaktionsmischung verteilt man den Rückstand zwischen Methylenchlorid und Wasser. trennt die organische Phase ab, entfernt das Lösungsmittel im Vakuum und kristallisiert den Rückstand aus Ethanol. Man erhält 18 g 4-(4-Chlorphenyl)-4-(4-dimethylaminophenyl)-2-ethylthio-5-oxo 2-pyrrolin-3-carbonitril in Form farbloser Kristalle: Fp. 195°C.

Das als Ausgangsprodukt verwendete 4-(4-Chlorphenyl)-5-(4-dimethyl aminophenyl)-5-hydroxy-2-thioxo-3-pyrrolin-3-carbonitril wird wie folgt hergestellt:

Eine Suspension von 60.0 g p-Dimethylamino-p-chlorbenzil. 19.5 g Cyanthioacetamid und 1 ml Piperidin in 1 l Dichlormethan wird 20 Stunden bei Raumtemperatur gerührt. Das ausgefallene Produkt wird abgesaugt, mit Dichlormethan ausgewaschen und aus Ethanol umkristallisiert. Man erhält 61.9 g gelbe Kristalle: Fp. 189° C (Zers.).

In analoger Weise erhält man folgende Verbindungen:

4-(4-Chlorphenyl)-(4-dimethylaminophenyl)-2-(morpholinoethylthio)-5-oxo-2-pyrrolin-3-carbonitril; Fp.215°C.

4-(4-Chlorphenyl)-4-(4-dimethylaminophenyl)-5-oxo-2-(2-piperidinoethylthio)-2-pyrrolin-3-carbonitril; Fp. 235°C (Zers.).

4-(4-Chlorphenyl)-2-(2-diethylaminoethylthio)-4-(4-dimethylaminophenyl)-5-oxo-2-pyrrolin-3-carbonitril,Fp. 196 - 198°C.

4-(4-Chlorphenyl)-2-(3-dimethylaminopropylthio)-4-(4-dimethylamino-phenyl)-5-oxo-2-pyrrolin-3-carbonitril: Fp. 180°C.

2-(2-Diethylaminoethylthio)-4(4-dimethylaminophenyl)-5-oxo-4-phenyl-2-pyrrolin-3-carbonitril; Fp 169°C.

4-(4-Dimethylaminophenylthio)-2-(2-morpholinoethylthio)-5-oxo-4-phenyl-2-pyrrolin-3-carbonitril; Fp. 209°C.

2-(2-Dimethylaminoethylthio)-4(4-dimethylaminophenyl)-5-oxo-4-2-pyrrolin-3-carbonitril;Fp. 172°C.

4-(4-Dimethylaminophenyl)-2-(2-piperidinoethylthio)-5-oxo-4-2-pyrrolin-3-carbonitril; Fp. 184°C.

4,4-Bis-(4-chlorphenyl)-2-(2-diethylaminoethylthio)-5-oxo-2-pyrrolin-3-carbonitril; Fp. 166°C.

2-(2-Diethylaminoethylthio)-4,4-bis-(4-methoxyphenyl)-5-oxo-2-pyrrolin-3-carbonitril: Fp. 164°C.

Die als Ausgangsprodukte verwendeten Verbindungen werden wie folgt hergestellt:

**5-Hydroxy-4,5-bis(4-methoxyphenyl)-2-thioxo-3-pyrrolin-3-carbonitril**

16,5 g 4,4-Dimethoxybenzil, 6,0 g Cyanthioacetamid und 15 Tropfen Piperidin werden 2 Stunden in 200 ml Chloroform am Wasserabscheider gekocht. Der ausgefallene gelbe Niederschlag wird abgesaugt, mit etwas Chloroform ausgewaschen und bei 80°C und 0,l Torr 12 Stunden getrocknet. Ausbeute 17,6 g gelbe Kristalle: Fp. 193°C (Zers.).

**4,5-Bis-(4-chlorphenyl)-5-hydroxy-2-thioxo-3-pyrrolin-3-carbonitril**

Eine Suspension von 22,3 g 4,4'-Dichlorbenzil, 6,5 g 2-Cyanthioace-tamid. 20 Tropfen Piperidin und 200 ml Chloroform wird 20 Stunden bei Raumtemperatur gerührt. Der ausgefallene gelbe Niederschlag wird abgesaugt, gut mit Chloroform ausgewaschen und getrocknet. Man erhält 22.0 g gelbe Kristalle: Fp. 213°C Zers.).

**5-(4-Dimethylaminophenyl)-5-hydroxy-4-phenyl-2-thioxo-3-pyrrolin-3carbonitril**

Eine Mischung von 10.1 g p-Dimethylaminobenzil, 3.2 g Cyanthioacetamid, 10 Tropfen Piperidin und 100 ml Ethanol wird 12 Stunden bei Raumtemperatur gerührt. Das ausgefallene Produkt wird abgesaugt. mit Chloroform ausgewaschen und aus Ethanol umkristallisiert. Ausbeute 6,0 g gelbe Kristalle: Fp. 184 185°C (Zers.).

**Beispiel 3**

**2-(2-Diethylaminoethylthio)-5-oxo-4-phenvl-4-(3-trifluormethylphenyl)pyrrolin-3-carbonitril**

19,5 g 3-Trifluormethylbenzil, 7,0 g Cyanthioacetamid, 0,5 ml Piperidin und 400 ml Chloroform wurden 20 Stunden bei Raumtemperatur gerührt. Die gelbe Suspension wurde auf ca. 100 ml eingeengt und der gelbe Niederschlag abgesaugt, ausgewaschen und getrocknet. Es wurden 24,0 g gelbes Produkt erhalten: Fp. 200 C (Zers.).

10,8 g gelbes Kondensationsprodukt und 8.3 g Kaliumcarbonat werden in 250 ml Ethanol 1 Stunde unter Rühren gekocht. Dann tropft man innerhalb von 10 Minuten eine Lösung von 5,2 g 2-Diethylaminoethyl-chlorid Hydrochlorid in 50 ml Ethanol hinzu, kocht noch eine halbe Stunde weiter und engt schließlich am Rotationsverdampfer ein.

Der Rückstand wird zwischen Methylenchlorid und Wasser verteilt und die organische Phase getrocknet. Nach Einengen erhält man 17,5 g ölige Base.

12,6 g Base werden in 350 ml Ether gelöst und langsam mit einer Lösung von 2,5 g Oxalsäure in 50 ml Ether versetzt. Der ausgefallene farblose Niederschlag wird abgesaugt und aus Ethanol umkristallisiert. Man erhält 7.6 g 2-(2-Diethylaminoethylthio)-5-oxo-4-phenyl-4-(3-tri-fluormethylphenyl)-2-pyrrolin-3-carbonitril in Form farbloser Kristalle: Fp. 126°C.

In analoger Weise erhält man 2-(2-Diethylaminoethylthio)-4-(4-fluor-phenyl)-5-oxo-4-phenyl-2-pyrrolin-3-carbonitril.

16,0 g 4-Fluorbenzil und 7,0 g Cyanthioacetamid ergeben 21,7 g gelbes Kondensationsprodukt vom Schmp. 170 - 185°C. 10,8 g Kondensationsprodukt ergaben nach Kristallisation der öligen Base aus Ethanol 11,6 g farblose Kristalle; Fp. 127°C.

**Beispiel 4**

**2-2-(Diethylaminoethylthio)-4-(4-dimethylaminophenyl)-5-oxo-4-(3-pyridyl-2-pyrolin-3-carbonîtril** (4a)

Man löst 2.3 g Natrium in 300 ml Ethanol, gibt 16.8 g 5-(4-Dimethyl-aminophenyl)-5-hydroxy-4-(3-pyridyl)-2-thioxo-2-pyrrolin-3-carbonitril hinzu und erhitzt 30 Minuten zum Sieden. Dann tropft man eine Lösung, von 8,6 g Diethylaminoethylchlorid Hydrochlorid in 50 ml Ethanol hinzu und kocht 1 Stunde weiter. Man engt ein, gibt zum öligen Rückstand Wasser und extrahiert mit Methylenchlorid. Die organische Phase wird eingeengt und der Rückstand aus Ethanol unter Zusatz von etwas Aktivkohle kristallisiert. Man erhält 14.6 g 2-(2-Diethylaminoethylthio)-4-(4-dimethylaminophenyl)-5-oxo-4-(3-pyridyl)-2-pyrrolin-3-carbonitril in Form farbloser Kristalle: Fp. 150 C

Das als Ausgangsprodukt verwendete 5-(4-Dimethylaminophenyl)-5-hy-droxy-4-(3-pyridyl)-2-thioxo-3-pyrrolin-3-carbonitril wird wie folgt hergestellt:.

Eine Suspension von 93.5 g 3-Pyridyl-4-dimethylaminophenylglyoxal. 35 g Cyanthioacetamid und 2 ml Piperidin in 1 l Methanol wird 20 Stunden bei Raumtemperatur gerührt. Der rote Niederschlag wird abgesaugt. gut mit Methanol ausgewaschen und aus Ethanol umkristallisiert. Ausbeute 105.6 g rote Kristalle: Fp. 191°C (Zers.).

In analoger Weise erhält man folgende Verbindungen:

4-(4-Dimethylaminophenyl)-2-(2-morpholinoethylthio)-5-oxo-4-(3-pyridyl)-2-pyrrolin-3-carbonitril; Fp. 149° C. (4b)

4-(4-Dimethylaminophenyl)-2-(3-dimethylaminopropylthio)-5-oxo-4-(3-pyridyl)-2-pyrrolin-3-carbonitril; Fp. 152° C. (4c)

4-(4-Dimethylaminophenyl)-2-(2-piperidinoethylthio)-5-oxo-4-(3-pyridyl)-2-pyrrolin-3-carbonitril; Fp. 180° C. (4d)

4-(4-Dimethylaminophenyl)-2-(2-Pyrrolidinoethylthio)-5-oxo-4-(3-pyridyl)-2-pyrrolin-3-carbonitril; Fp. 196° C. (4e)

4-(4-Dimethylaminophenyl)-5-oxo-2-(diisopropylaminoethylthio)-4-(3-pyridyl)-2-pyrrolin-3-carbonitril; Fp. 157° C. (4f)

2-(2-Dimethylaminoethylthio)-4-(4-dimethylaminophenyl)-5-oxo-4-(3-pyridyl)-2-pyrrolin-3-carbonitril; Fp. 190° C. (4g)

4-(4-Dimethylaminophenyl)-5-oxo-2-propylthio-4-(3-pyridyl)-2-pyrrolin-3-carbonitril; Fp. 193° C. (4h)

4-(4-Dimethylaminophenyl)-2-methylthio-5-oxo-4-(3-pyridyl)-2-pyrrolin-3-carbonitril; Fp. 206° C. (4i)

4-(4-Dimethylaminophenyl)-2-(3-morpholinopropylthio)-5-oxo-4-(3-pyridyl)-2-pyrrolin-3-carbonitril; Fp. 172° C. (4j)

2-(3-Diethylaminopropylthio)-4-(4-dimethylaminophenyl)-5-oxo-4-(3-pyridyl)-2-pyrrolin-3-carbonitril; Fp. 134° C. (4k)

4-(4-Dimethylaminophenyl)-5-oxo-2-(2-piperazinoethylthio)-4-(3-pyridyl)-2-pyrrolin-3-carbonitril; Oxalat; Fp. 130° C. (4l)

4-(4-Dimethylaminophenyl)-5-oxo-2-(3-piperidinopropylthio)-4-(3-pyridyl)-2-pyrrolin-3-carbonitril; Fp. 168° C. (4m)

2-Ethylthio-4-(4-dimethylaminophenyl)-5-oxo-4-(3-pyridyl)-2-pyrrolin-3-carbonitril; Fp = 213° C. (4n)

2-(2-Aminoethylthio)-4-(4-dimethylaminophenyl)-5-oxo-4-(3-pyridyl)-2-pyrrolin-3-carbonitril; Fp = 205° - 206° C.

(4o)
2-(3-Aminopropylthio)-4-(4-dimethylaminophenyl)-5-oxo-4-(3-pyridyl)-2-pyrrolin-3-carbonitril; Fp = 205° - 208° C, zers. (4p)

**Beispeil 5**

**2-(2-Diethylaminoethylthio)-4-(4-fluorphenyl)-5-oxo-4-(3-pyridyl)-2pyrrolin-3-carbonitril-Hydrochlorid**

27,2 g 4-Fluorphenyl-3-pyridylglyoxal. 10.0 g Cyanthioacetamid. 10 Tropfen Piperidin und 500 ml Chloroform werden 6 Stunden bei Raumtemperatur gerührt. Das ausgefallene gelbe Produkt wird abgesaugt, mit etwas Chloroform ausgewaschen und bei 60 C getrocknet. Ausbeute 28,2 g gelbes Produkt: Fp. 170 - 172 C (Zers.).

Man löst 2.2 g Natrium in 250 ml absolutem Ethanol, gibt 15,0 g Kondensationsprodukt aus 4-Fluorphenyl-3-pyridylglyoxal und Cyanthioacetamid hinzu und erhitzt 15 Minuten zum Sieden. Nun tropft man eine Lösung von 8.3 g Diethylaminoethylchlorid Hydrochlorid in 50ml Ethanol innerhalb von 15 Minuten hinzu und erhitzt danach noch 4 Stunden am Rückfluß. Nach dem Abkühlen wird das Lösungsmittel abgezogen und der Rückstand zwischen Chloroform und Wasser verteilt. Die getrocknete Chloroformphase wird zur Trockene eingeengt, der ölige Rückstand in absolutem Ether/Ethanol aufgenommen und die Lösung mit trockenem Chlorwasserstoff gesättigt. Ein zu Beginn ausgefallener Niederschlag geht nach kurzer Zeit wieder in Lösung. Das Lösungsmittel wird abgezogen und der Rückstand mit Ether verrieben. Das erstarrte Produkt wird aus Isopropanol und schließlich nochmals aus Ethanol unter Zusatz einiger Tropfen Wasser kristallisiert. Man erhält 5. 1 g 2-(2-Diethylaminoethylthio)-4-(4-fluorphenyl)-5-oxo-4-(3-pyridyl)-2-pyrrolin-3-carbonitril Hydrochlorid in Form farbloser Kristalle; Fp. 247°C.

In analoger Weise erhält man die folgenden Verbindungen:

**2-(2-Diethylaminoethylthio)-4-(4-methoxyhenyl)-5-oxo-4-(3-pyridyl)2-pyrrolin-3-carbonitril**

12,0 g 4-Methoxyphenyl-3-pyridylglyoxal und 5,0 g Cyanthioacetamid ergaben 15,8 g orangefarbenes Produkt, Fp. 200°C (Zers.). 10,0 g Kondensationsprodukt ergaben nach Kristallisieren aus Methanol 7,8 g farblose Kristalle: Fp. 133°C.

**2-(2-Diethylaminoethylthio)-5-oxo-4-phenyl-4-(3-pyridyl)-2-pyrrolin-3carbonitril**

20.0 g Phenyl-3-pyridylglyoxal und 9.5 g Cyanthioacetamd ergaben 23,7 g gelbes Kondensationsprodukt; Fp. 140° C Zers.). 11,7 g Kondensationsprodukt ergaben nach Kristallisieren aus Ethanol 10.5 g farblose Kristalle; Fp. 133°C.

**2-(2-Diethylaminoethylthio)-4-(3,4-dimethoxyphenyl)-5-oxo-4-(3-pyridyl) -2-pyrrolin-3-carbonitril**

24.5 g 3.4-Dimethoxyphenyl-3-pyridylglyoxal und 8.8 g Cyanthioacetamid ergaben 30.0 g gelbes Kondensationsprodukt: Fp. 175 - 180°C (Zers.) - (Ethanol). 15, 0 g Kondensationsprodukt ergaben nach Kristallisieren aus Isopropanol 10,5 g farblose Kristalle: Fp. 133 - 135 °C.

**2-(2-Diethylaminoethylthio)-5-oxo-4-(3-pyridyl)-4-(3-trifluormethylphenyl)-2-pyrrolin-3-carbonitrit-Oxatlat**

22.9 g 3-Pyridyl-3-trifluormethylphenylglyoxal und 7.0 g Cyanthioacetamid ergaben nach 12 Stunden Reaktionszeit 19.3 g gelbes Kondensationsprodukt; Fp. 216 C (Zers.). 19.0 g Kondensationsprodukt ergaben 14.0 g ölige Base, die in etherischer Lösung als Oxalat gefällt wurde. Ausbeute 14.2 g farblose Kristalle: Fp. 115°C (Zers.) (Ethanol).

**Beispiel 6**

**2-(2-Diethylaminoethylthio)-5-oxo-4-phenyl-4-(2-thienyl)-2-pyrrolin-3carbonitril** (6a)

29,5 g Benz-2-thenyl, 13.4 g Cyanthioacetamid, 0.5 ml Piperidin und 500 ml Methylenchlorid werden 20 Stunden bei Raumtemperatur gerührt. Das ausgefallene Produkt wird abgesaugt und aus Ethanol kristallisiert. Man erhält 29.6 g grünliche Kristalle. Fp. 187 - 189° C. 14,9 g Kondensationsprodukt aus Benz-2-thenyl und Cyanthioacetamid werden zusammen mit 13,8 g Kaliumcarbonat in 300 ml Ethanol 1 Stunde unter Rühren am Rückfluß erhitzt. Man tropft innerhalb von 10 Minuten eine Lösung von 8,6 g 2-Diethylaminoethylchlorid Hydrochlorid in 100 ml Ethanol hinzu und kocht 45 Minuten weiter. Man engt die Reaktionsmischung ein, versetzt mit Wasser und extrahiert mit Methylenchlorid. Die organische Phase wird eingeengt und der Rückstand aus Ethanol kristallisiert. Man erhält 15.3 g 2-(2-Diethyl-aminoethylthio)-5-oxo-4-phenyl-4-(2-thienyl)-2-pyrrolin-3-carbonitril in Form farbloser Kristalle: Fp. 165° C.

In analoger Weise erhält man folgende Verbindungen:
2-(2-Ethylthio)-5-oxo-4-phenyl-4-(2-thienyl)-2-pyrrolin-3-carbonitril: Fp. 115° C. (6b)
2- (2-morpholinoetylthio)-5-oxo-4-phenyl-4-(2-thienyl) -2-pyrrolin-3-carbonitril: Fp. 185 - 186° C. (6c)
2-(2-piperidinoethylthio)-5-oxo-4-phenyl-4- (2-thienyl) -2-pyrrolin-3-carbonitril: Fp. 180° C. (6d)
2-(3-Diethylaminopropylthio)-5-oxo-4-phenyl-4-(2-thienyl)-2-pyrrolin-3-carbonitril: Fp. 151° C. (6e)
5-oxo-4-phenyl-2- (2-pyrrolidinethylio)-4-(2-phenyl) -2-pyrrolin-3-carbonitril. (6f)

**Beispiel 7**

**2-(3-Dimethylaminopropylthio)-5-oxo-4,4-bis(2-eyridyl)-2-pyrrolin-3-carbontril**

10.2 g 5-Hydroxy-4,5-bis (2-pyridyl)-2-thioxo-3-pyrrolin-3-carbonitril und 8.4 g wasserfreies Kaliumcarbonat werden 30 Minuten in 250 ml Ethanol unter Rückfluß erhitzt. Man tropft eine Lösung von 4,8 g 3-Dimethylaminopropylchlorid-Hydrochlorid in 30 ml Ethanol hinzu und kocht 1 Stunde weiter. Man engt am Rotationsverdampfer ein, versetzt den Rückstand mit Wasser und extrahiert mit Methylenchlorid. Die organische Phase wird über Natriumsulfat getrocknet. das Lösungsmittel wird abgezogen und der ölige Rückstand aus Ethanol kristallisiert. Man erhält in 63 %iger Ausbeute 2-(3-Dimethylaminopropylthio)-5-oxo-4.4-bis(2-pyridyl)-2-pyrrolin-3-carbonitril als farblose Kristalle: Fp. 184 - 185° C.

Das als Ausgangsprodukt verwendete 5-Hydroxy-4,5-bis(2-pyridyl)-2-thioxo-3-pyrrolin-3-carbonitril wird wie folgt hergestellt:

Man löst 11.0 g 2.2'-Pyridyl und 5,0 g Cyanthioacetamid in 200 ml absolutem Ethanol. gibt 5 Tropfen Piperidin hinzu und erhitzt eine halbe Stunde unter Rückfluß. Man kühlt auf 0° C ab. saugt den ausgefallenen Niederschlag ab, wäscht mit etwas Ethanol aus und kristallisiert aus Ethanol um. Die Kristalle werden bei 100° C und 0.1 Torr. 12 Stunden getrocknet. Ausbeute 9.8 g: Fp. 188° C (Zers.)

In analoger Weise werden folgende Verbindungen erhalten:
5-oxo-2-(2-piperidinoethylthio) 4,4-bis (2-pyridil) -2-pyrrolin-3-carbonitril; Fp. 203 - 204° C.
2-(2-Diethylaminoethylthio)-5-oxo-4,4-bis-(2-pyridyl)-2-pyrrolin-3-carbonitril: Fp. 135 - 136° C.
2-(2-Ethylthio)-5-oxo-4.4-bis-(2-pyridyl)-2-pyrrolin-3-carbonitril: Fp. 180° C.

**Beispiel 8**

**4-Diphenyl-2-isopropylthio-5-oxo-2-pyrrolin-3-carbonitril**

Man löst 1.2 g Natrium in 150 ml Ethanol, gibt 15.2 g 5-Hydroxy-4.5-diphenyl-2-thioxo-3-pyrrolin-3-carbonitril hinzu und erhitzt 20 Minuten zum Sieden. Innerhalb von 10 Minuten tropft man sodann eine Lösung von 7.0 g Isopropylbromid in 25 ml absolutem Ethanol hinzu und erhitzt weitere 3 Stunden am Rückfluß. Nach dem Abkühlen wird das Lösungsmittel am Rotationsverdampfer abgezogen und der Rückstand mit 100 ml Wasser verrieben. Der Niederschlag wird abgesaugt und aus Ethanol kristallisiert. Man erhält 11.1 g (64%) 4,4-Diphenyl-2-isopropylthio-5-oxo-2-pyrrolin-3-carbonitril in Form farbloser Kristalle: Fp. 160° C.

Das als-Zwischenprodukt entstehende 5-oxo-4,4-diphenyl-2-thioxopyrro-liden-3-carbonitril der Formel IV wird wie folgd hergestellt:

13,8 g 5-Hydroxy-4,5-diphenyl-2-thioxo-3-pyrrolin-3-carbonitril werden 15 Minuten in 300 ml Ethanol mit 12.4 g Kaliumcarbonat unter Rühren gekocht. Die anfangs tiefgelbe Lösung wird bald hell und nahezu farblos.

Nach dem Abkühlen gießt man die Suspension unter Rühren in 1 1 Eiswasser. säuert mit verdünnter Salzsäure vorsichtig an. Der ausgefallene Niederschlag wird abgesaugt und aus Toluol umkristallisiert. Ausbeute 10.4 g gelbliche Kristalle. Fp. 170° C (Zers.).

**Beispiel 9**

**4,4-Diplhenyl-2-isopropylthio-5-oxo-2-pyrrolin-3-carbonitril**

Man löst 1,2 g Natrium in 150 ml Ethanol, gibt 15,2 g 5-Hydroxy-4,5-diphenyl-2-thioxo-3-pyrrolin-3-carbonitril hinzu und erhitzt 20 Minuten zum Sieden. Innerhalb von 10 Minuten tropft man sodann eine Lösung von 7,0 g Isopropylbromid in 25 ml abs. Ethanol hinzu und rückflussiert weitere 3 Stunden. Nach dem Abkühlen wird das Lösungsmittel am Rotavapor abgezogen und der Rückstand mit 100 ml Wasser verrieben. Der Niederschlag wird abgesaugt und aus Ethanol kristallisiert. Man erhält 11,1 g (64,0%) farblose Kristalle vom Schmelzpunkt 160°C.

Analog wurde 2-Butylthio-4,4-diphenyl-5-oxo-2-pyrrolin-3-carbonitril erhalten, Ausbeute 76 %.

**Beispiel 10**

**4,4-Diphenyl-2-(1-methyl-propylthio)-5-oxo-2-pyrrolin-3-carbonitril**

Man löst 0,8 g Natrium in 150 ml abs. Ethanol gibt 10,0 g 5-Hydroxy-4,5-diphenyl-2-thioxo-3-pyrrolin-3-carbonitril hinzu und erhitzt 30 Minuten zum Sieden. Danach entfernt man das Lösungsmittel am Rotavapor und nimmt den Rückstand in 100 ml Dimethylformamid auf. Man gibt 5,1 g sek. Butylbromid hinzu, rührt 24 Stunden bei Raumtemperatur, gibt dann nochmals 3,0 g Alkylierungsmittel hinzu und rührt nochmals 24 Stunden. Danach wird das Lösungsmittel im Wasserstrahlvakuum abdestilliert und der Rückstand mit Wasser verrieben. Der Niederschlag wird abgesaugt, mit Wasser ausgewaschen und aus Ethanol kristallisiert. Man erhält 7,0 g farblose Kristalle vom Schmelzpunkt 155° - 156°C.

**Patentansprüche für die Vertragsstaaten BE CH DE FR GB IT U LU NL SE**

1.) Verbindungen der allgemeinen Formel I

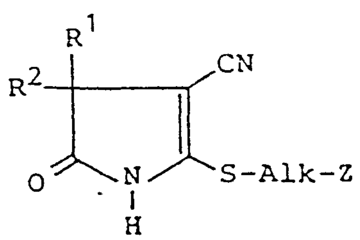

in welcher $R^1$ und $R^2$ gleich oder verschieden sin und einen unsubstituierten oder durch bis zu zwei Halogenatome-, $C_{1-4}$ Alkoxygruppen, $C_{1-4}$ ODialkylaminogruppen oder Trifluormethylgruppen substituierten phenyl -, pyridyl - oder Thienylring, Alk eine geradkettige oder verzweiqte C -Alkylenkette und Z Wasserstoff darstellt, wobei für den Fall, daß Alk eine geradkettige Alkylenkette bedeutet, Z auch eine Aminogruppe der allgemeinen Formel 11 bedeuten kann,

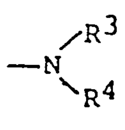

in welcher $R^3$ und $R^4$ gleich oder verschieden sind und ein Wasserstoffatom oder einen Methyl- Athyl-, mit dem Stickstoffatom an das sie gebunden sind. eine Pyrrolidino-, Piperidino-. Morpholino- oder Piperazinogruppe darstellen können,

sowie deren pharmakologisch verträgliche Salze.

2.) 2-(2-Diethylaminoethylthio)-4-(4-dimethylaminophenl) - 5-oxo-4-(3-pyridyl)-2-pyrrolin-3-carbonitril

3.) Verfahren zur Herstellung von Verbindungen der allgemeinen Formel I,

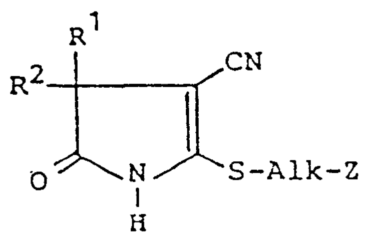

in welcher die Reste $R^1$, $R^2$, Alk und Z die in anspruch 1 genannte Bedeutung haben, sowie von deren pharmakologisch verträglichen Salzen, dadurch gekennzeichnet, daß man eine Verbindung der allgemeinen Formel III

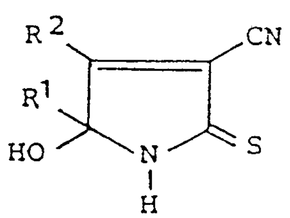

in welcher $R^1$ und $R^2$ die oben genannte Bedeutung haben, in einem Lösungsmittel mit einer Base zu einer Verbindung der allgemeinen Formel IV

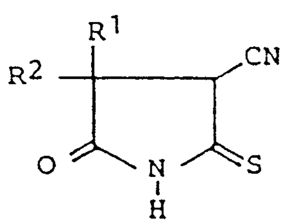

in welcher $R^1$ und $R^2$ die oben genannte Bedeutung haben, umlagert und diese anschließend mit einer Verbindung der allgemeinen Formel V X-Alk-Z (V)

in welcher Z und Alk die obengenannte Bedeutung haben und X eine reaktive Ester oder Ethergruppe datstellt,, umsetzt und die erhaltene Verbindung der allgemeinen Formel I anschließend gegebenenfalls in deren pharmakologisch verträglichen Salze überführt.

4.) Verfahren gemäß Anspruch 3, dadurch gekennzeichnet. daß man ein polares Lösungsmittel verwendet.

5.) Verfahren gemäß Anspruch 3 bis 4, dadurch gekennzeichnet, daß man als Lösungsmittel Methanol, Ethanol oder n-Butanol verwendet.

6.) Verfahren gemäß Anspruch 3 bis 5, dadurch gekennzeichnet. daß man als Base Kaliumhydroxid. Kaliumcarbonat. Natriummethanolat. Natriumethanolat oder Kalium-tert.-butylat verwendet.

7.) Arzneimittel enthaltend mindestens eine Verbindung gemäß Anspruch I bis 2 und geeignete pharmazeutische Hilfs- und Trägerstoffe.

**Patentansprühe für den Vertragsstaat AT**

1.) Verfahren zur Herstellung von Verbindungen der allgemeinen Formel 1,

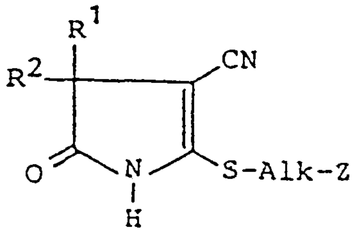

in welcher $R^1$ und $R^2$ gleich oder verschieden sind und einen unsubstituierten oder durch bis zu zwei Halogenatome-, $C_{1-4}$ Alkoxygruppen, $C_{1-4}$ Dialkylaminogruppen oder Trifluormethylgruppen substituierten Phenyl-, Pyridyl- oder Thienylring, Alk eine geradkettige oder verzweigte $C_{1-4}$ -Alkylenkette und Z Wasserstoff darstellt, wobei für den Fall, daß Alk eine geradkettige Alkylenkette bedeutet, Z auch eine Aminogruppe der allgemeinen Formel II bedeuten kann.

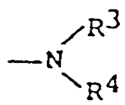

in welcher $R^3$ und $R^4$ gleich oder verschieden sind und ein Wasserstoffatom oder einen Methyl- Athyl-, Propyl- oder Isopropylrest bedeuten oder zusammen mit dem Stickstoffatom an das sie gebunden sind. eine Pyrrolidino-, Piperidino-. Morpholino- oder Piperazinogruppe darstellen können, sowie von deren pharmakologisch verträglichen Salzen dadurch gekennzeichnet daß man eine Verbindung der allgemeinen Formel III

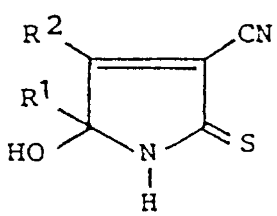

in welcher $R^1$ und $R^2$ die oben genannte Bedeutung haben, in einem Lösungsmittel mit einer Base zu einer Verbindung der allgemeinen Formel IV

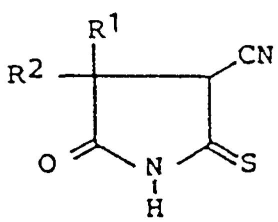

in welcher $R^1$ und $R^2$ die oben genannte Bedeutung haben, umlagert und diese anschließend mit einer Verbindung der allgemeinen Formel V X-Alk-Z (V),

in welcher Z und Alk die obengenannte Bedetung haben und X eine reaktive Ester- oder Ethergruppe darstellt umsetzt und die erhaltene Verbindung der allgemeinen Formel I anschließend gegebenenfalls in deren pharmakologisch verträglichen Salze überführt.

2.) Verfahren nach Anspruch 1 zur Herstellung von 2-(2-Diethylamino-ethylthio)-4-(4-dimethylaminophenyl)-5-oxo-4-(3-pyridyl)-2-pyrrolin-3-carbonitril.

3.) Verfahren gemäß Ansprüchen 1 - 2. dadurch gekennzeichnet daß man ein polares Lösungsmittel verwendet.

4.) Verfahren gemäß Ansprüchen 1 bis 3. dadurch gekennzeichnet, daß man als Lösungsmittel Methanol. Ethanol oder n-Butanol verwendet.

5.) Verfahren gemäß Ansprüchen 1 bis 4. dadurch gekennzeichnet, daß man als Base Kaliumhydroxid, Kaliumcarbonat. Natriummethanolat. Natriumethanolat oder Kalium-tert.butylat verwendet.

**Claims**

1.) Compounds of the general formula:

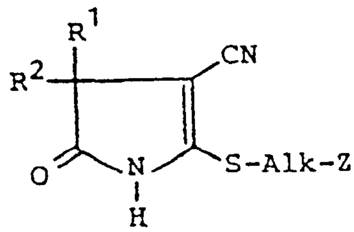

wherein $R^1$ and $R^2$, which are the same or different, are phenyl, pyridyl or thienyl rings which are unsubstituted or substituted by up to two halogen atoms, $C^{1\text{-}4}$ alkoxy radicals, $C^{1\text{-}4}$ dialkylamino radicals or trifluoromethyl radicals, Alk is a straight-chained or branched $C^{1\text{-}4}$ alkylene chain and Z is a hydrogen atom, with the proviso that when Alk is a straight-chained alkylene chain, Z can also be an Amino radical of the general formula II:

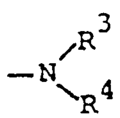

in which $R^3$ and $R^4$ are the same or different and are hydrogen atoms or methyl, ethyl, propyl or isopropyl radicals or, together with the nitrogen atom to which they are attached can also form a pyrrolidino, piperidino, morpholino or piperazino radical and the pharmacologically salts thereof.

2.) 2-(2-Diethylaminoethylthio)-4-(4-dimethylaminophenyl)-5-oxo-4-(3-pyridyl)-2-pyrrolin-3-carbonitril.

3.) Process for the preparation of compounds according to the general formula I:

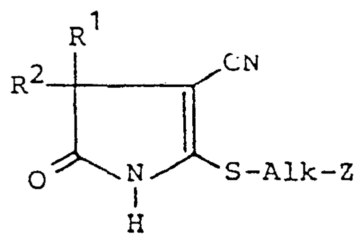

wherein the radicals $R^1$, $R^2$, Alk and Z have the meaning of Claim 1, and of the pharmacolcgically acceptable salts thereof, characterized in that a compound of the general formula III:

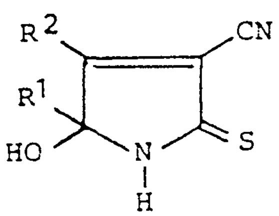

in wich $R^1$ and $R^2$ have the above meanings is rearranged in a solvent with a base to give a compound of the general formula IV,

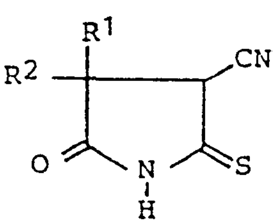

in which $R^1$ and $R^2$ have the same meanings as in Claim 1, and this is subsequently reacted with a compound of the general formula V: X-Alk-Z (V), in which Z and Alk have the same meanings as in Claims 1, and X is a reactive ester or ether group, whereafter, if desired, the product obtained is converted into a pharmacologically acceptable salt.

4.) Process according to Claim 3, wherein a polar solvent is employed.

5.) Process according to Claim 3 to 4, wherein the polar solvent is methanol, ethanolor n-butanol.

6.) Process according to any of Claims 3 to 5, wherein the base used is potassium hydroxide, potassium carbonate, sodium methanolate, sodium ethanolate or potassium tert.-butylate.

7.) Pharmaceutical compositions, comprising at least one compound according to Claim 1 to 2 and an appropriate pharmaceutical carrier and/or adjuvant.

**Claims for the contracting state: AT**

1.) Process for the preparation of compounds of the general formula:

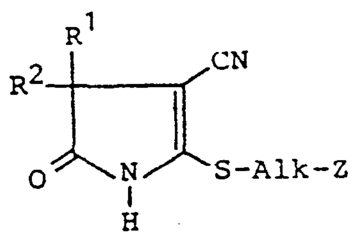

wherein $R^1$ and $R^2$, which are the same or different, are phenyl, pyridyl or thienyl rings which are unsubstituted or substituted by up to two halogen atoms, $C_{1-4}$ alkoxy radicals, $C_{1-4}$ dialkylamino radicals or trifluoromethyl radicals, Alk is a straight-chained or branched $C_{1-4}$ alkylene chain and Z is a hydrogen atom, with the proviso that when Alk is a straight-chained alkylene chain, Z can also be an Amino radical of the general formula II:

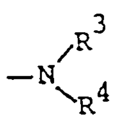

in which $R^3$ and $R^4$ are the same or different and are hydrogen atoms or methyl, ethyl, propylor isopropyl radicals or, together with the nitrogen atom to which they are attached, can also form a pyrrolidino, piperidino, morpholino or piperazino radical and of the pharmacologically acceptable salts thereof characterized in that a compound of the general formula III:

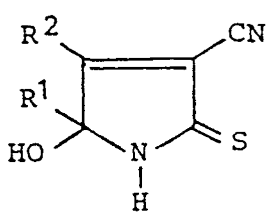

wherein $R^1$ and $R^2$ have the above meanings is rearranged in a solvent with a base to give a compound of the general formula IV.:

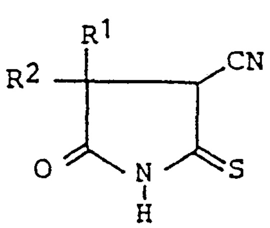

in which $R^1$ and $R^2$ have the same meanings as above, and this is subsequently reacted with a compound of the general formula V.: X-Alk-Z in which Z and Alk have the above meanings, and X is a reactive ester or ether group, whereafter, if desired, the product obtained is converted into a pharmacologically acceptable salt.

2.) Process according to Claim 1 for the manufacture of 2-(2-diethylamino-ethylthio)-4-(4-dimethylaminophenyl)-5-oxo-4-(3-pyridyl)-2-ayrroline-3-carbonitril.

3.) Process according to Claims 1 to 2, wherein a polar solvent is employed.

4.) Process according to Claims 1 to 3, wherein the polar solvent is methanol, ethanol or n-butyl.

5.) Process according to Claims 1 to 4, wherein the base used is potassium hydroxide, potassium carbonate, sodium methanolate, sodium ethanolate or potassium tert. butylate.

**Revendications**

1.- Composés de formule générale I:

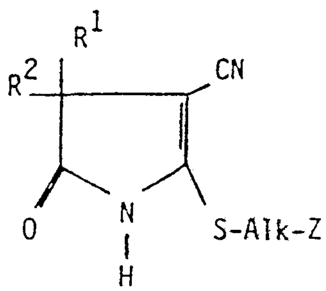

dans laquelle:

$R^1$ et $R^2$ sont identiques ou différents et représentent un phényle, pyridyle ou thiényle non substitué ou substitué par jusqu'à 2 atomes d'halogéne, groupes alkoxy en $C_1$ à $C_4$ groupes dialkylamino en $C_1$ à $C_4$ ou groupes trifluorométhyles, Alk représente une chaîne alkylée en $C_1$ à $C_4$ droite ou ramifiée; et représente un atome d'hydrogène, Z, dans le cas où Alk représente une chaîne alkylène droite, pouvant également représenter un groupe amino de formule générale II,

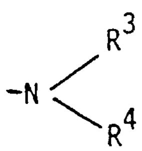

formule II dans laquelle $R^3$ et $R^4$ sont identiques ou différents, et représentent un atome d'hydrogène ou un reste méthyle, éthyle, propyle ou isopropyle ou peuvent former ensemble, en même temps avec l'atome d'azote auquel ils sont rattachés, un groupe pyrrolidino, pipéridino, morpholino ou pipérazino, ainsi que leurs sels pharmaceutiquement acceptables.

2.- 2-(2-diéthylaminoéthylthio)-4-(4-diméthylaminophényl)-5-oxo-pyridyl)-2-pyrrolino-3-carbonitrile.

3.- Procédé de préparation de composés de formule générale I:

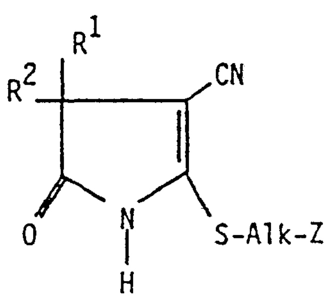

dans laquelle les restes $R^1$,$R^2$, Alk et Z ont les significations indiquées dans la revendication 1, ainsi que leurs sels pharmaceutiquement acceptables, caractérisé en ce que, dans un solvant et en présence d'une base, on transpose un composé de formule générale III:

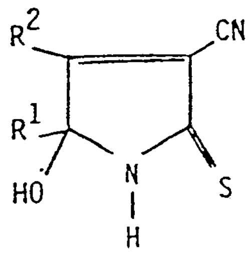

dans laquelle $R^1$ et $R^2$ ont les significations indiquées ci-dessus, en un composé de formule générale IV:

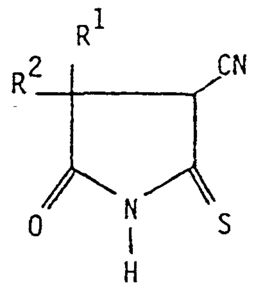

dans laquelle $R^1$ et $R^2$ ont les significations indiquées ci-dessus, et on fait ensuite réagir ce dernier avec un composé de formule géné rale V: X-Alk-Z (V)

dans laquelle: Z et Alk ont les significations indiquées ci-dessus; et X représente un groupe réactif ester ou éther, et l'on transforme le composé obtenu de formule générale I en un se pharmaceutiquement acceptable.

4.- Procédé selon la revendication 3, caractérisé en ce que l'on utilise un solvant polaire.

5.- Procédé selon les revendications 3 ou 4, caractérisé en ce que l'on utilise comme solvant le méthanol, l'éthanol et le n-butanol.

6.- Procédé selon les revendications 3 à 5, caractérisé en ce que l'on utilise comme base l'hydroxyde de potassium, le carbonate de potassium, le méthanolate de sodium, éthanolate de sodium ou le tertioutylate de potassium.

7.- Médicament contenant au moins un composé selon les revendications 1 à 2, et des adjuvants et supports pharmaceutiques appropriés.

**Revendications pour l'Etat contractan: AT**

1.- Procédé de préparation de composés de formule générale:

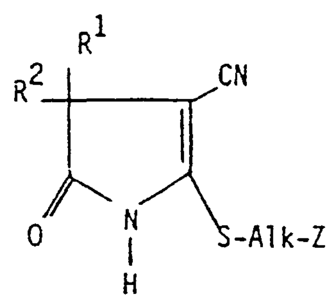

dans laqulle:

$R^1$ et $R^2$ sont identiques ou différents et représentent un phényle, pyridyle ou thiényle non substitué ou substitué par jusqu'à 2 atomes d'halogéne, groupes alkoxy en $C_1$ à $C_4$, groupes dialkylamino en $C_1$ à C4 ou

groupes trifluorométhyles, Alk représente une chaine alkyléne droite ou ramifiée en $C_1$ à $C_4$; et Z représente un atome d'hydrogène, Z, dans le cas où Alk représente une chaîne alkyléne droite, pouvant également représenter un groupe amino de formule générale II,

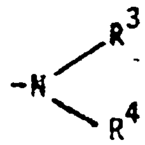

formule II dans laquelle $R^3$ et $R^4$ sont identiques ou differérents, et représentent un atome d'hydrogène ou un reste méthyle, éthyle, propyle ou isopropyle ou forment, en même temps avec l'atome d'azote auquel ils sont rattachés, un groupe pyrrolidino, pipéridino, morpholino ou pipérazino, ainsi que leurs sels pharmaceutiquement acceptables, caractérisé en ce que dans un solvant et en présence d'une base, on transpose un composé de formule générale III:

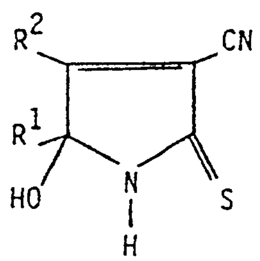

dans laquelle $R^1$ et $R^2$ ont les significations indiquées ci-dessus, en un composé de formule générale IV:

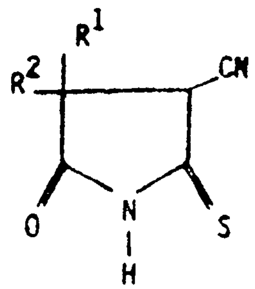

dans laquelle $R^1$ et $R^2$ ont les significations indiquées ci-dessus, et on fait ensuite réagir ce composé avec un composé de formule générale V: X-Alk-Z dans laquelle: Z et Alk ont les significations indiquées ci-dessus; et X représente un groupe réactif ester ou éther, et l'on transforme ensuite le composé de formule générale I obtenu, le cas échéant, en un sel pharmaceutiquement acceptable.

2.- Procédé selon la revendication 1, de préparation de 2-(2-diéthylaminoéthylthio)-4-(4-diméthylaminophényl)-5-oxo-4-(3-pyridyl) 2-pyrrolino-3-carbonitrile.

3.- Procédé selon les revendications 1 et 2, caractérisé en ce que l'on utilise un solvant polaire.

4.- Procédé selon les revendications 1 à 3, caractérisé en ce que l'on utilise comme solvant du méthanol, de l'éthanol et du n-butanol.

5.- Procédé selon les revendications 1 à 4, caractérisé en ce que l'on utilise comme base de l'hydroxyde de potassium, du carbonate de potassium, du méthanolate de sodium, de l'éthanolate de sodium ou du tertiobutylate de potassium.